# EUROPEAN PATENT APPLICATION

(11) **EP 4 079 318 A1**
(43) Date of publication of application: **26.10.2022**
(21) Application number: 21382349.5
(22) Date of filing: 22.04.2021
(51) Int. Cl.: A61K 39/00, A61P 35/00, C07K 14/725, C12N 5/0783, C07K 16/28

(54) **COMBINATION OF CAR-NK CELLS WITH NKG2A BLOCKING AGENTS, PHARMACEUTICAL COMPOSITION COMPRISING THE SAME AND USE THEREOF**

(71) Applicant: Fundación del Sector Público Estatal Centro Nacional de Investigaciones Oncológicas Carlos III (F.S.P. CNIO), 28029 Madrid (ES); Fundación para la Investigación Biomédica del Hospital Universitario 12 de Octubre, 28041 Madrid (ES)
(72) Inventor: Martínez López, Joaquín, 28041 Madrid (ES); Valeri Lozano, Antonio, 28041 Madrid (ES); García Ortiz, Almudena, 28041 Madrid (ES); Gallardo Delgado, Miguel, Madrid 28029 (ES); Encinas Mayoral, Jessica, 28041 Madrid (ES); Maroto Martín, Elena, 28041 Madrid (ES); Castellano Esparza, Eva, 28041 Madrid (ES)
(74) Representative: Manuel Illescas y Asociados, S.L.

(57) **Abstract**

The present invention refers to a pharmaceutical composition comprising Natural Killer (NK) cells transduced with a chimeric antigen receptor (CAR-NK cells), and at least one NKG2A blocking agent and its use as a medicament. The present invention also refers to a pharmaceutical composition comprising CAR-NK cells for use in the treatment of cancer, wherein said composition is administered before, simultaneously with, or after administering at least one NKG2A blocking agent.

## Description

### TECHNICAL FIELD

The present invention relates to cancer therapies based on Natural Killer (NK) cells, genetically modified with a chimeric antigen receptor (CAR-NK cells), combined with NKG2A blocking agents.

### BACKGROUND ART

A chimeric antigen receptor (CAR) is an artificially modified fusion protein that consists of an extracellular antigen recognition domain fused to an intracellular signaling domain. T cells genetically modified with a CAR (CAR-T cells) have been widely used in the treatment of cancer and exhibit improved tumor-specific targeting and cytotoxicity against cancer cells.

Natural Killer (NK) cells genetically modified with a CAR (CAR-NK cells) also exhibit improved tumor-specific targeting and cytotoxicity against cancer cells, which make them suitable for the treatment of cancer, similarly to CAR-T cells. There are important benefits associated to the use of CAR-NK cells in cell-based cancer immunotherapy. In particular, allogeneic NK cells can be used as effector cells since they are not responsible for graft versus host disease (GVHD), because they do not require HLA matching. Furthermore, CAR-NK cells do not usually induce cytokine storms.

On the other hand, one strategy used in the state of the art to improve NK cell activity against tumor cells is the therapeutic blockade of NKG2A, an immune checkpoint receptor generally expressed on NK cells and upregulated on cells from many cancer types. The humanized anti-NKG2A antibody monalizumab is an example of NKG2A blocking agent used to improve the anti-tumor response mediated by NK cells, and other anti-NKG2A antibodies are under development. Nevertheless, the combination of NKG2A blocking agent with CAR-NK therapies has not become available in the state of the art until now.

In the past years, several cancer immunotherapies based on CAR-NK cells have been developed. Despite the progress made in this field, there is still a need to develop improved CAR-NK cancer therapies.

### SUMMARY OF INVENTION

As used herein, "Chimeric Antigen Receptor" (CAR) refers to a synthetic receptor consisting of at least an extracellular antigen-binding domain, at least a hinge domain, at least a transmembrane domain, and at least an intracellular signaling domain derived from the T cell receptor (TCR), most commonly CD3ζ (the zeta chain associates with the T cell receptor complex). CAR may also include one or two co-stimulatory domains, usually derived from CD28 and/or 4-1BB.

As used herein, "transducing" refers to a process by which an exogenous polynucleotide is introduced into a host cell. A "transduced" cell is one which has been transduced with an exogenous polynucleotide. The transduced cell includes the primary subject transduced cell and its transduced progeny.

As used herein, "NKG2A" refers to a C-type lectin inhibitory checkpoint receptor. NKG2A belongs to the NKG2 family, also known as CD159 (Cluster of Differentiation 159). NKG2A is generally expressed on NK cells and on T cells. NKG2A is upregulated on cells from many cancer types.

As used herein, "HLA-E" refers to a nonclassical HLA class I molecule, which interacts with NKG2C (activatory receptor) and with higher affinity for NKG2A (inhibitory receptor) on NK cells and T cells. HLA-E is over-expressed in different cancer types, as acute myeloid leukemia (AML), breast cancer or colorectal cancer (Wieten et al., 2014) and it has been proposed as a mechanism of tumors to escape from immune surveillance (Seliger et al., 2016).

As used herein, "NKG2D" refers to a C-type lectin receptor. NKG2D belongs to the NKG2 family, also known as CD159 (Cluster of Differentiation 159). NKG2D is generally expressed on NK cells and on T cells. NKG2D is a receptor that recognizes the following NKG2D ligands: MHC class I chain related protein A (MICA), MHC class I chain related protein B (MICB), and the structurally diverse unique long 16 binding proteins 1 to 6 (ULBP1-6). NKG2D ligands are commonly overexpressed in various tumors.

As used herein, "BCMA" refers to B-cell maturation antigen, also known as tumor necrosis factor receptor superfamily member 17 (TNFRSF17). BCMA is a member of the TNF-receptor superfamily. BCMA is preferentially expressed in mature B lymphocytes and is important for B cell development and autoimmune response.

As used herein, "α5β1" refers to an integrin that binds to matrix macromolecules and proteinases and thereby stimulates angiogenesis. α5β1 is the primary receptor for fibronectin.

As used herein, "αVβ3" refers to a type of integrin that is a receptor for vitronectin, expressed by platelets. αVβ3 is a receptor for phagocytosis on macrophages or dendritic cells.

As used herein, "APRIL" refers to a proliferation-inducing ligand (APRIL), also known as tumor necrosis factor ligand superfamily member 13 (TNFSF13). APRIL is a protein of the TNF superfamily recognized by the cell surface receptor TACI. APRIL is a ligand for BCMA.

As used herein, "BAFF" refers to B-cell activating factor (BAFF), also known as tumor necrosis factor ligand superfamily member 13B or CD257. BAFF is a cytokine that belongs to the tumor necrosis factor (TNF) ligand family. BAFF is a ligand for receptors TACI, BCMA, and BAFF-R. BAFF is expressed in B cell lineage cells, and acts as a potent B cell activator. It has been also shown to play an important role in the proliferation and differentiation of B cells.

As used herein, "carbonic anhydrase IX" refers to a transmembrane dimeric metalloenzyme with an extracellular active site that facilitates acid secretion in the gastrointestinal tract. Carbonic anhydrase IX is overexpressed in many types of cancer.

As used herein, "CD123" refers to cluster of differentiation 123, also known as interleukin 3 receptor alpha (IL-3Rα).

As used herein, "CD19" refers to cluster of differentiation 19. In humans, CD19 is expressed in all B lineage cells.

As used herein, "CD20" refers to cluster of differentiation 20. CD20 is expressed on the surface of all B-cells beginning at the pro-B phase (CD45R+, CD117+) and progressively increasing in concentration until maturity.

As used herein, "CD28" refers to cluster of differentiation 28. CD28 is expressed on T cells and provide co-stimulatory signals required for T cell activation and survival.

As used herein, "CD30" refers to cluster of differentiation 30, also known as TNFRSF8. CD30 is a cell membrane protein of the tumor necrosis factor receptor family and tumor marker. CD30 is expressed by activated, but not by resting, T and B cells.

As used herein, "CD33" refers to cluster of differentiation 33, also known as Siglec-3, gp67 and p67. CD33 is a transmembrane receptor expressed on cells of myeloid lineage.

As used herein, "CD52" refers to cluster of differentiation 52. CD52 is present on the surface of mature lymphocytes, but not on the stem cells from which these lymphocytes were derived. CD52 is also found on monocytes and dendritic cells.

As used herein, "CEA" refers to carcinoembryonic antigen. It encompasses a set of highly related glycoproteins involved in cell adhesion.

As used herein, "c-MET" refers to tyrosine-protein kinase Met or hepatocyte growth factor receptor (HGFR). c-MET is a single pass tyrosine kinase receptor essential for embryonic development, organogenesis and wound healing.

As used herein, "EpCAM" refers to epithelial cell adhesion molecule, a transmembrane glycoprotein mediating Ca²⁺-independent homotypic cell-cell adhesion in epithelia.

As used herein, "EphA3" refers to EPH receptor A3 (ephrin type-A receptor 3).

As used herein, "ErbB1" refers to epidermal growth factor receptor, also known as EGFR or HER1.

As used herein, "ErbB2" refers to receptor tyrosine-protein kinase ErbB2, also known as CD340 or HER2. Amplification or over-expression of ErbB2 has been shown to play an important role in the development and progression of certain aggressive types of breast cancer.

As used herein, "ErbB3" refers to receptor tyrosine-protein kinase ErbB3, also known as HER3.

As used herein, "FAP" refers to fibroblast activation protein alpha. FAP is selectively expressed in reactive stromal fibroblasts of epithelial cancers, granulation tissue of healing wounds, and malignant cells of bone and soft tissue sarcomas.

As used herein, "folate binding protein" is a protein that bind folate. Folate binding protein is over-expressed in ovarian and endometrial cancers, as well as in breast, lung, colorectal, and renal cell carcinomas.

As used herein, "GD2" refers to disialoganglioside 2.

As used herein, "GD3" refers to disialoganglioside 3.

As used herein, "GM2" refers to ganglioside monosialic 2.

As used herein, "gpA33" refers to cell surface glycoprotein A33.

As used herein, "HMW-MAA" refers to chondroitin sulfate proteoglycan 4.

As used herein, "IGF1R" refers to insulin-like growth factor 1 receptor. IGF1R is a transmembrane receptor that is activated by a hormone called insulin-like growth factor 1 (IGF-1) and by a related hormone called IGF-2.

As used herein, "IL-11Rα" refers to interleukin 11 receptor, alpha subunit.

As used herein, "IL-13Rα2" refers to interleukin-13 receptor subunit alpha-2, also known as CD213A2.

As used herein, "mesothelin" is a protein expressed in mesothelial cells.

As used herein, "MUC1" refers to mucin 1.

As used herein, "MUC16" refers to mucin 16.

As used herein, "PSCA" refers to prostate stem cell antigen, a glycosylphosphatidylinositol-anchored cell membrane glycoprotein.

As used herein, "PSMA" refers to prostate-specific membrane antigen.

As used herein, "RANKL" refers to receptor activator of nuclear factor kappa-B ligand.

As used herein, "ROR1" is a tyrosine-protein kinase transmembrane receptor, also known as neurotrophic tyrosine kinase, receptor-related 1 (NTRKR1).

As used herein, "TAG-72" refers to tumor-associated glycoprotein 72, found on the surface of many cancer cells.

As used herein, "tenascins" are extracellular matrix glycoproteins. Tenascins are abundant in the extracellular matrix of developing vertebrate embryos. Tenascins are present around healing wounds and in the stroma of some tumors.

As used herein, "TRAIL-R1" refers to death receptor DR4 (TRAIL-R1 receptor). TRAIL refers to a cytokine that is produced and secreted by most normal tissue cells, which causes apoptosis primarily in tumor cells, by binding to certain death receptors.

As used herein, "TRAIL-R2" refers to death receptor DR5 (TRAIL-R2 receptor).

As used herein, "VEGF" refers to vascular endothelial growth factor. VEGF is a signal protein produced by cells that stimulates the formation of blood vessels. VEGF is involved in both vasculogenesis (the de novo formation of the embryonic circulatory system) and angiogenesis (the growth of blood vessels from pre-existing vasculature).

As used herein, "VEGFR" refers to receptors for vascular endothelial growth factor (VEGF).

As used herein, "expanded NK cells" refers to NK cells which has been cultured *ex vivo* with agents that induce NK cells proliferation or expansion. Examples of agents that induce proliferation or expansion of NK cells are cytokines such as IL-2.

As used herein, "activated NK cells" refers to NK cells which has been cultured *ex vivo* with agents that stimulate and activate NK cells, inducing NK cell proliferation or expansion, NK cell cytokine production, and NK cell cytotoxic activity. Examples of agents that activate NK cells are cytokines such as IL-2.

As used herein, "effective amount" refers to the quantity of a pharmaceutical composition, cells, and/or an NKG2A blocking agent, that provides an objectively identifiable improvement in the subject's condition, recognized by a qualified observer, wherein said patient has been treated with said quantity of the pharmaceutical composition, cells and/or NKG2A blocking agent. In examples, an effective amount of CAR-NK cells, and of NKG2A blocking agents are used in an amount sufficient to treat or inhibit cancer in a subject. In examples, an effective amount is an amount sufficient to reduce or ameliorate one or more symptoms of cancer in a subject.

The technical problem to be solved is to provide improved cancer immunotherapies based on CAR-NK cells.

The invention as defined in the claims provides a solution to this technical problem.

The present invention is based on the combination of Natural Killer (NK) cells transduced with a chimeric antigen receptor (CAR-NK cells), with at least one NKG2A blocking agent, which provides an effective cancer therapy. The effective cancer therapy based on this combination is synergistic.

The present invention provides a pharmaceutical composition comprising NK cells transduced with a chimeric antigen receptor (CAR-NK cells), and at least one NKG2A blocking agent.

There are various methods of transducing NK cells with a CAR, in which exogenous polynucleotides are introduced into a host cell. Physical treatments, chemical compounds, biological particles or combinations thereof can be used in said methods of introducing exogenous polynucleotides into a host cell.

Methods of introducing exogenous polynucleotides into a host cell may include, but are not limited to: electroporation, microinjection, gene gun, cell squeezing, impalefection, sonoporation, optical transfection, hydrodynamic delivery, lipofection, magnetofection, particle bombardment and RNA transfection.

Methods of introducing exogenous polynucleotides into a host cell can include the use of DNA or RNA vectors. Vectors for introducing exogenous polynucleotides into a host cell may include but are not limited to: transposon-based vectors, *Sleeping Beauty* transposon-based vectors and minicircles.

As used herein, "minicircles" refers to supercoiled minimal expression vectors developed for application in non-viral gene delivery. They are derived from their parental plasmids via an intramolecular recombination process, during which the majority of bacterial backbone sequences are depleted from the vector. The minicircles are, therefore, significantly reduced in size, and, as a consequence, they have been shown to enhance gene delivery into a variety of cell lines.

Chemical compounds for introducing exogenous polynucleotides into a host cell can include, but are not limited to: calcium phosphate, highly branched organic compounds, such as dendrimers, cationic polymers, such as DEAE-dextran or polyethylenimine (PEI), liposomes and polymeric gene carriers (polyplexes).

Biological particles for introducing exogenous polynucleotides into a host cell include viral particles may include, but are not limited to: lentivirus, retrovirus, poxvirus, adenovirus, adeno-associated virus and herpes simplex virus I.

The pharmaceutical composition of the invention may consist of water solutions, saline solutions or solutions buffered to physiological pH.

The pharmaceutical composition of the invention may be administered to a subject in several different ways, depending on whether the treatment is local or systemic, and depending on the area to be treated. Thus, for example, the pharmaceutical composition of the invention may be administered to a subject by ocular, vaginal, rectal, intranasal, oral, by inhalation, or by parenteral route, whether intradermal, subcutaneous, intramuscular, intraperitoneal, intrarectal, intra-arterial, intralymphatic, intravenous, intrathecal, intra-ocular, intracranial, intraventricular, intratracheal and intratumoral. Parenteral administration, if used, is generally performed by injection. The solutions for injection can be prepared in various ways, such as solutions or liquid suspensions.

The pharmaceutical composition of the invention can be administered with or without other active ingredients.

The pharmaceutical composition of the invention can be administered in single or multiple doses.

In a preferred embodiment, the pharmaceutical composition of the invention comprises two vials, wherein a vial comprises CAR-NK cells and wherein the other vial comprises at least one NKG2A blocking agent.

The present invention also provides the pharmaceutical composition of the invention for use as a medicament.

In addition, the present invention provides a method of treatment of a subject, comprising administering before, simultaneously with, or after administration of an effective amount of at least one NKG2A blocking agent, an effective amount of a pharmaceutical composition comprising CAR-NK cells.

The present invention also provides a pharmaceutical composition comprising CAR-NK cells for use in the treatment of cancer, wherein said composition is administered before, simultaneously with, or after administration of at least one NKG2A blocking agent.

In an embodiment, the present invention refers to a method of treatment of cancer in a subject, comprising administering before, simultaneously with, or after administration of an effective amount of at least one NKG2A blocking agent, an effective amount of a pharmaceutical composition comprising CAR-NK cells.

In another embodiment, the present invention refers to the use of a pharmaceutical composition comprising CAR-NK cells for the manufacture of a medicament for the treatment of cancer, wherein said pharmaceutical composition is administered before, simultaneously with, or after administration of at least one NKG2A blocking agent.

In a preferred embodiment of the pharmaceutical composition for use of the invention, the cancer is selected from the group consisting of B cell acute lymphoblastic leukemia (ALL), minimal residual disease-positive ALL, chronic lymphatic leukemia (CLL), hairy cell leukemia, non-Hodgkin's lymphoma, B cell lymphoma, mantle cell lymphoma (MCL), diffuse large B cell lymphoma (DLBCL), follicular lymphoma, marginal zone B cell lymphoma, acute myeloid leukemia (AML), chronic myeloid leukemia (CML), Burkitt's lymphoma, lymphoplasmacytic lymphoma, myelodysplastic syndrome (MDS), multiple myeloma, breast cancer, colorectal cancer, ovarian cancer, rectal cancer, stomach cancer, testicular cancer, anal region cancer, uterine cancer, colon cancer, rectal cancer, renal-cell carcinoma, liver cancer, non-small cell lung cancer, small intestine cancer, esophagus cancer, melanoma, Kaposi's sarcoma, endocrine system cancer, thyroid gland cancer, parathyroid gland cancer, adrenal gland cancer, bone cancer, pancreatic cancer, skin cancer, head cancer, neck cancer, brain stem glioma, pituitary adenoma, epidermoid cancer, cervix squamous cell cancer, fallopian tubes carcinoma, endometrium carcinoma, vagina carcinoma, soft tissue sarcoma, urethra cancer, vulva carcinoma, penis cancer, bladder cancer, kidney cancer, ureter cancer, renal pelvis carcinoma, spinal axis tumor, central nervous system (CNS) neoplasm, primary CNS lymphoma, and tumor angiogenesis.

In another preferred embodiment, the pharmaceutical composition is for use in the treatment of multiple myeloma of a subject who has relapsed or progressed. Said subject has relapsed/progressed after treatment with chemotherapy, immunotherapy or any conventional multiple myeloma treatment known in the art.

In yet another embodiment of the pharmaceutical composition for use of the invention, the cancer comprises HLA-E overexpressing cells.

In a preferred embodiment of the pharmaceutical composition or the invention, or the pharmaceutical composition for use of the invention, the CAR specifically recognizes a target antigen selected from the group consisting of α5β1, αVβ3, APRIL, BAFF, BCMA, carbonic anhydrase IX, CD123, CD19, CD20, CD28, CD30, CD33, CD52, CEA, c-MET, EpCAM, EphA3, ErbB1, ErbB2, ErbB3, FAP, folate binding protein, GD2, GD3, GM2, gpA33, HMW-MAA, IGF1R, IL-11Rα, IL-13Rα2, Lewis-Y2, mesothelin, MUC1, MUC16, NKG2D ligands, PSCA, PSMA, RANKL, ROR1, TAG-72, tenascins, TRAIL-R1, TRAIL-R2, VEGF, and VEGFR.

In another preferred embodiment of the pharmaceutical composition of the invention, or the pharmaceutical composition for use of the invention, the CAR is NKG2D-CAR or BCMA-CAR.

In yet another preferred embodiment of the pharmaceutical composition of the invention, or the pharmaceutical composition for use of the invention, the CAR-NK cells are activated and expanded.

In a further preferred embodiment of the pharmaceutical composition of the invention, or the pharmaceutical composition for use of the invention, the at least one NKG2A blocking agent is an anti-NKG2A antibody.

Several anti-NKG2A antibodies have been disclosed in the prior art, including, but not limited, to monalizumab, BMS-986315 (Bristol-Myers Squibb) and antibody clone Z199 (Beckman Coulter).

Therefore, in a more preferred embodiment of the pharmaceutical composition of the invention, or the pharmaceutical composition for use of the invention, the anti-NKG2A antibody is selected from the group consisting of monalizumab, BMS-986315 and antibody clone Z199.

In a further preferred embodiment of the pharmaceutical composition of the invention, or the pharmaceutical composition for use of the invention, the pharmaceutical composition further comprises at least one pharmaceutically acceptable excipient or carrier.

The excipient may be an inert ingredient or ingredients, including, but not limited to: thickeners, buffers, preservatives, antioxidants, metal chelating agents, isotonicity agents, surfactants, nanoparticles, liposomes and others. The pharmaceutical composition may include further active ingredients.

Pharmaceutically acceptable carriers include sterile aqueous solutions or dispersions and sterile powders for the extemporaneous preparation of sterile injectable solutions or dispersion. The use of such pharmaceutically acceptable carriers is known in the art. As a way of example, the carrier may be a diluent.

As used in this specification and the appended claims, the singular forms "a", "an", and "the" include plural referents unless the content clearly dictates otherwise.

Unless defined otherwise, all the technical and scientific terms have the same meaning as those commonly understood by a person skilled in the art in the field of the invention.

Throughout the description and the claims, the terms "comprises", "comprising" and their variants are not limiting in nature and therefore do not aim to exclude other technical features.

Throughout the description and the claims, the terms "comprise", "comprising" and their variants include, specifically, the term "consisting" or "consisting of".

### BRIEF DESCRIPTION OF DRAWINGS

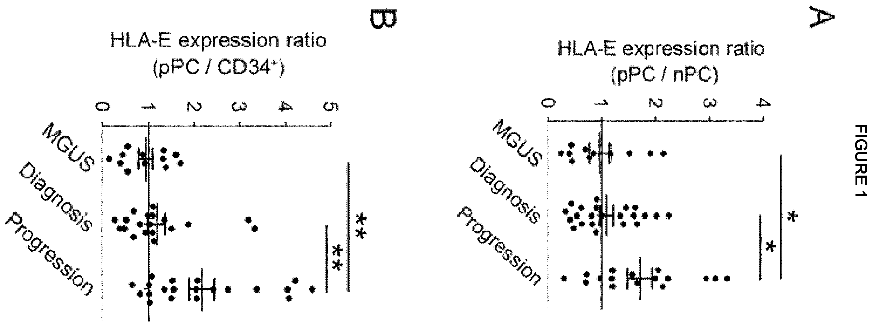

**Figure 1****.** HLA-E is over-expressed in symptomatic multiple myeloma (MM) patient samples. **A.** Flow cytometry analysis of HLA-E expression ratios between pathologic plasma cells (pPC) and normal plasma cells (nPC) from monoclonal gammopathy of undetermined significance (MGUS) or symptomatic MM patient samples at diagnosis or progression. The mean ± standard error is shown. **B.** Flow cytometry analysis of HLA-E expression ratios between pPC and CD34⁺ cells from MGUS or symptomatic MM patient samples at diagnosis or progression. The mean ± standard error is shown.

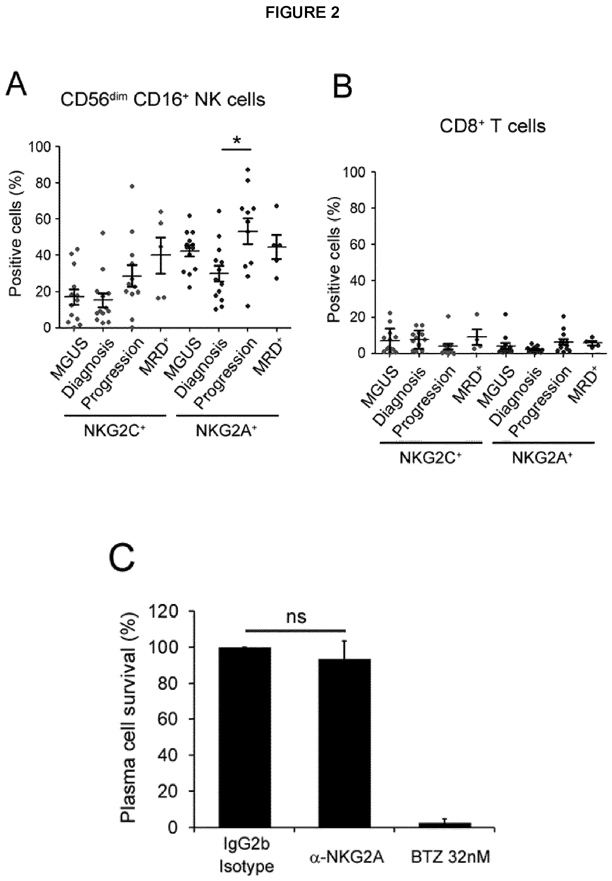

**Figure 2****.** Analysis of NKG2C and NKG2A expression in bone marrow (BM) infiltrated CD56^{dim} CD16⁺ cytotoxic NK cells and CD8⁺ T cells from MM patient samples. Percentage analysis by flow cytometry of NKG2C⁺ and NKG2A⁺ cells in CD56^{dim} CD16⁺ cytotoxic NK cells **(A)** and CD8⁺ T cells **(B)** from MGUS or MM patient samples at diagnosis, progression or MRD+ status. The mean ± standard error is shown. **C.** Flow cytometry analysis of primary plasma cell survival after the 48 hour-culture of bone marrow mononuclear cells isolated from MM patient samples treated with bortezomib (BTZ), the α-NKG2A antibody (Clone Z199, Beckman Coulter) or its corresponding IgG2b isotype control. ns: not significant.

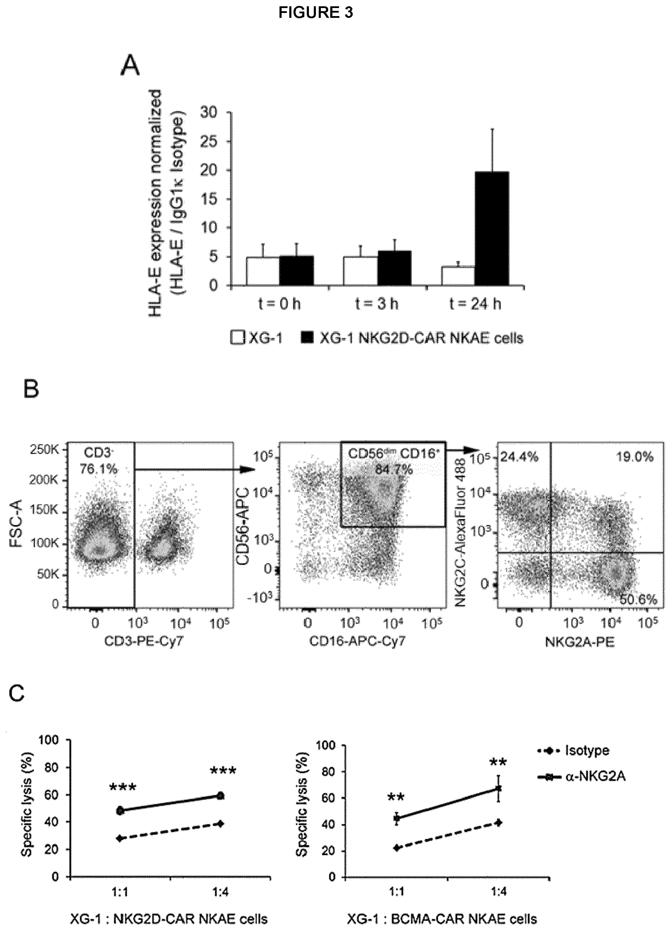

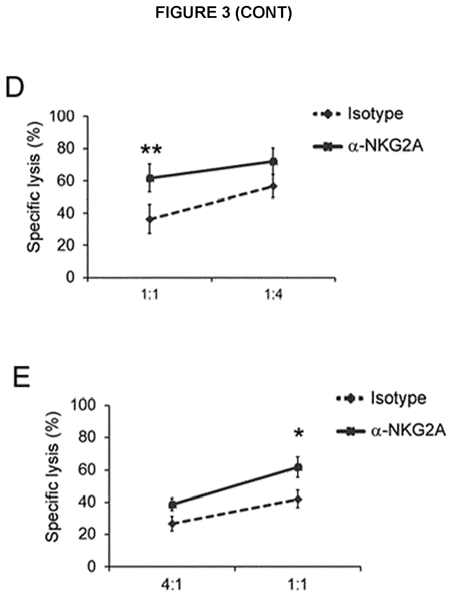

**Figure 3****.** NKG2A blocking increases CAR-NK cytotoxic activity against XG-1 and ARP-1 MM cell lines and NALM-6 B-cell lymphoblastic leukemia (B-ALL) cell line. **A.** Flow cytometry analysis of HLA-E expression in XG-1 alone or in co-culture with NKG2D-CAR activated and expanded NK cells (NKAE cells) for the times indicated. The mean ± standard error is shown (n=3). **B.** Representative flow cytometry density plots illustrating NKG2C/NKG2A expression in NKG2D-CAR NKAE cells generated from one of the MM patients. **C.** Specific lysis against XG-1 MM cell line of NKG2D-CAR (left) or BCMA-CAR (right) NKAE cells treated with α-NKG2A antibody (Clone Z199, Beckman Coulter) or its corresponding IgG2b isotype control analyzed by 3h-Calcein-AM release assay. The mean ± standard error is shown (n=3). **p<0.01; ***p<0.001. **D.** Specific lysis against ARP-1 MM cell line of NKG2D-CAR NKAE cells treated with α-NKG2A antibody (Clone Z199, Beckman Coulter) or its corresponding IgG2b isotype control analyzed by 3h-Calcein-AM release assay. The mean ± standard error is shown (n=3). **p<0.01. **E.** Specific lysis against NALM-6 B-ALL cell line of NKG2D-CAR NKAE cells treated with α-NKG2A antibody (Clone Z199) or its corresponding IgG2b isotype control analyzed by 3h-Calcein-AM release assay. *p<0.05.

### DESCRIPTION OF EMBODIMENTS

### Example 1. HLA-E expression in multiple myeloma (MM) pathologic plasma cells (pPC)

The analysis of HLA-E expression levels in MM patient samples showed that this biomarker is over-expressed in pathologic plasma cells (pPC) with regard to normal plasma cells (nPC) in 55% of MM symptomatic patients, achieving higher values in patients in progression, in which the HLA-E over-expression includes 75% of patients (**Figure 1A** and **Table 1**)**.** Table 1 shows percentages of patients with pPC/nPC HLA-E expression ratios >, < or equal to 1.

| **Table 1. HLA-E expression ratio (pPC/nPC)** | | | |
|---|---|---|---|
| | Patients ratio > 1 | Patients ratio = 1 | Patients ratio < 1 |
| Symptomatic MM (diagnosis and progression) | 55% (n = 21) | 5% (n = 2) | 40% (n = 15) |
| Progression | 75% (n = 12) | 6% (n = 1) | 19% (n = 3) |

It was also detected that HLA-E is over-expressed in pPC respect to CD34⁺ cells, as another control healthy population, in 65% of MM symptomatic patients and 81% of patients in progression (**Figure 1B** and **Table 2**). Table 2 shows percentages of patients with pPC/CD34⁺ HLA-E expression ratios >, < or equal to 1.

| **Table 2. HLA-E expression ratio (pPC/CD34⁺)** | | | |
|---|---|---|---|
| | Patients ratio > 1 | Patients ratio = 1 | Patients ratio < 1 |
| Symptomatic MM (diagnosis and progression) | 65% (n = 26) | 10% (n = 4) | 25% (n = 10) |
| Progression | 81% (n = 17) | 9.5% (n = 2) | 9.5% (n = 2) |

The expression of HLA-E receptors NKG2A and NKG2C in bone marrow (BM) infiltrated T and cytotoxic NK cells from MM patient samples was analyzed. It has been observed that there is a higher percentage of BM infiltrated CD56^{dim} CD16⁺ cytotoxic NK cells expressing NKG2A than NKG2C, especially in patients in progression **(****Figure 2A****).** By contrast in CD8⁺ T cells, NKG2A expression does not seem to be statistically relevant to inhibit the cytotoxic activity of BM infiltrated T cells **(****Figure 2B****).** The treatment with α-NKG2A antibody (Clone Z199) does not reduce the plasma cell population present in isolated MM bone marrow mononuclear cells. Therefore, α-NKG2A antibody monotherapy is not capable of restoring the cytotoxic activity of MM bone marrow infiltrated NK cells **(****Figure 2C****)** as it can be seen with the comparison to the IgG2b isotype control. The IgG2b isotype control is an antibody that lacks specificity to NKG2A but matches the class/type of the α-NKG2A antibody (IgG2b). The IgG2b isotype control is used to determine the unspecific recognition of NKG2A by the constant region of the α-NKG2A antibody.

### Example 2. Blocking of NKG2A on NKG2D-CAR or BCMA-CAR NK cells

In this example, it has been assessed whether pPC HLA-E expression increases conferring resistance to MM cells or B-ALL in a CAR-NK immunotherapy. For this purpose, NK Activated and Expanded cells (NKAE cells) were generated from peripheral blood (PB) healthy donor samples by co-culture of peripheral blood mononuclear cells from healthy donors with irradiated (100 Gy) K562-mb21-41BBL cell line in the presence of IL-2 (50 IU/ml), cytokine maintained during all the culture. At day 8 NKAE cells were transduced with CAR lentiviral particles in retronectin-coated plates with the chimeric antigen receptor (CAR) NKG2D or BCMA-CAR, obtaining NKG2D-CAR or BCMA-CAR NKAE cells. Co-cultures between XG-1 MM cell line (a MM NK resistant cell line which express high levels of HLA-E) and NKG2D-CAR NKAE cells were performed and the HLA-E expression levels at 3 and 24 hours were studied by flow cytometry. In these conditions, HLA-E expression increased at 24 hours, suggesting a potential role for this molecule in the CAR therapy resistance **(****Figure 3A****).** The NKG2C and NKG2A expression was analyzed in NKAE CAR cells **(****Figure 3B****).** Co-cultures between XG-1 or ARP-1 MM cell lines, or NALM-6 B-ALL cell line, and NKG2D-CAR NKAE cells were performed. The α-NKG2A antibody blocking treatment significantly improved the CAR cytotoxicity increasing NK cells cytolytic activity over XG-1 either of NKG2D-CAR NKAE cells or BCMA-CAR NKAE cells **(****Figure 3C****)** or ARP-1 **(****Figure 3D****)** MM cell lines, or over NALM-6, a B-ALL cell line **(****Figure 3E****),** as it can be seen with the comparison to the IgG2b isotype control (same IgG2b isotype control as used in Example 1).

### CITATION LIST

Seliger et al. (2016). HLA-E expression and its clinical relevance in human renal cell carcinoma. Oncotarget, 7(41), 67360-67372. doi:10.18632/oncotarget.11744
Wieten et al. (2014). Clinical and immunological significance of HLA-E in stem cell transplantation and cancer. Tissue Antigens, 84(6), 523-35. doi:10.1111/tan.12478

## Claims

1. A pharmaceutical composition comprising Natural Killer (NK) cells transduced with a chimeric antigen receptor (CAR-NK cells), and at least one NKG2A blocking agent.

2. The pharmaceutical composition according to claim 1, comprising two vials, wherein a vial comprises CAR-NK cells and wherein the other comprises at least one NKG2A blocking agent.

3. The pharmaceutical composition according to claim 1 or 2, for use as a medicament.

4. A pharmaceutical composition comprising CAR-NK cells for use in the treatment of cancer, wherein said composition is administered before, simultaneously with, or after the administration of at least one NKG2A blocking agent.

5. The pharmaceutical composition for use according to claim 4, wherein the cancer is selected from the group consisting of B cell acute lymphoblastic leukemia (ALL), minimal residual disease-positive ALL, chronic lymphatic leukemia (CLL), hairy cell leukemia, non-Hodgkin's lymphoma, B cell lymphoma, mantle cell lymphoma (MCL), diffuse large B cell lymphoma (DLBCL), follicular lymphoma, marginal zone B cell lymphoma, acute myeloid leukemia (AML), chronic myeloid leukemia (CML), Burkitt's lymphoma, lymphoplasmacytic lymphoma, myelodysplastic syndrome (MDS), multiple myeloma, breast cancer, colorectal cancer, ovarian cancer, rectal cancer, stomach cancer, testicular cancer, anal region cancer, uterine cancer, colon cancer, rectal cancer, renal-cell carcinoma, liver cancer, non-small cell lung cancer, small intestine cancer, esophagus cancer, melanoma, Kaposi's sarcoma, endocrine system cancer, thyroid gland cancer, parathyroid gland cancer, adrenal gland cancer, bone cancer, pancreatic cancer, skin cancer, head cancer, neck cancer, brain stem glioma, pituitary adenoma, epidermoid cancer, cervix squamous cell cancer, fallopian tubes carcinoma, endometrium carcinoma, vagina carcinoma, soft tissue sarcoma, urethra cancer, vulva carcinoma, penis cancer, bladder cancer, kidney cancer, ureter cancer, renal pelvis carcinoma, spinal axis tumor, central nervous system (CNS) neoplasm, primary CNS lymphoma, and tumor angiogenesis.

6. The pharmaceutical composition for use according to any one of claims 3 to 5, in the treatment of multiple myeloma of a subject who has relapsed or progressed.

7. The pharmaceutical composition for use according to any one of claims 3 to 6, wherein the cancer comprises HLA-E overexpressing cells.

8. The pharmaceutical composition according to claim 1 or 2, or the pharmaceutical composition for use according to any one of claims 3 to 7, wherein the CAR specifically recognizes a target antigen selected from the group consisting of α5β1, αVβ3, APRIL, BAFF, BCMA, carbonic anhydrase IX, CD123, CD19, CD20, CD28, CD30, CD33, CD52, CEA, c-MET, EpCAM, EphA3, ErbB1, ErbB2, ErbB3, FAP, folate binding protein, GD2, GD3, GM2, gpA33, HMW-MAA, IGF1R, IL-11Rα, IL-13Rα2, Lewis-Y2, mesothelin, MUC1, MUC16, NKG2D ligands, PSCA, PSMA, RANKL, ROR1, TAG-72, tenascins, TRAIL-R1, TRAIL-R2, VEGF, and VEGFR.

9. The pharmaceutical composition according to claim 1 or 2, or the pharmaceutical composition for use according to any one of claims 3 to 8, wherein the CAR is NKG2D-CAR or BCMA-CAR.

10. The pharmaceutical composition according to claim 1 or 2, or the pharmaceutical composition for use according to any one of claims 3 to 9, wherein the CAR-NK cells are activated and expanded.

11. The pharmaceutical composition according to claim 1 or 2, or the pharmaceutical composition for use according to any one of claims 3 to 10, wherein the at least one NKG2A blocking agent is an anti-NKG2A antibody.

12. The pharmaceutical composition, or the pharmaceutical composition for use, according to claim 11, wherein the anti-NKG2A antibody is selected from the group consisting of monalizumab, BMS-986315 and antibody clone Z199.

13. The pharmaceutical composition according to claim 1 or 2, or the pharmaceutical composition for use according to any one of claims 3 to 12, further comprising at least one pharmaceutically acceptable excipient or carrier.
